# EUROPEAN PATENT APPLICATION

(11) **EP 4 640 309 A2**
(43) Date of publication of application: **29.10.2025**
(21) Application number: 23907941.1
(22) Date of filing: 14.12.2023
(51) Int. Cl.: B01J 19/00, B67B 7/00

(54) **MOBILE INDUSTRIAL DEVICE FOR MANUFACTURING DETERGENTS**

(30) Priority: 21.12.2022 SA 122440914
(71) Applicant: Shebily, Abdulaziz, Jeddah 23337 (SA)
(72) Inventor: Shebily, Abdulaziz, Jeddah 23337 (SA)
(74) Representative: Viering, Jentschura & Partner mbB Patent- und Rechtsanwälte
(86) International application number: PCT/SA2023/050016
(87) International publication number: WO 2024/136702

(57) **Abstract**

This device is a small, portable, self-contained manufacturing system that functions as a miniature factory. It produces cleaning agents from raw materials. The chemical proportions are adjusted based on the type and quantity of the desired product. The device determines and combines these proportions based on chemical formulas inputted through the programming language used during its manufacturing. Upon the user selecting the type and quantity of the required detergent, the device manufactures and dispenses the finished product, ready for use, without any direct human handling of chemicals (as shown in Figure A1).

## Description

### Background of the Invention:

This invention relates to a small, portable, self-contained manufacturing system that functions as a miniature factory. It produces cleaning agents from raw materials. The chemical proportions are adjusted based on the type and quantity of the desired product. The device determines and combines these proportions based on chemical formulas inputted through the programming language used during its manufacturing. Upon the user selecting the type and quantity of the required detergent, the device manufactures and dispenses the finished product, ready for use, without any direct human handling of chemicals (as shown in Figure A1). The key difference between this device (Mobile Detergent Manufacturing Factory) and the American invention documented under No. (202024103 1US) is that the American automated solution device relies on pre-made solutions in their final form to achieve laboratory results, analyze samples, and achieve the intended purpose using ready-made materials. In contrast, this Mobile Detergent Manufacturing Factory operates on the principle of manufacturing and producing cleaning agents by combining raw chemical materials in their initial state, ultimately producing a finished product ready for use. It incorporates the correct chemical formulation principles to transform raw materials into a final product suitable for consumer use in regular cleaning.

### General Description of the Invention:

This invention relates to a small, portable, self-contained manufacturing system that functions as a miniature factory. It produces cleaning agents from raw materials. The chemical proportions are adjusted based on the type and quantity of the desired product. The device determines and combines these proportions based on chemical formulas inputted through the programming language used during its manufacturing. Upon the user selecting the type and quantity of the required detergent, the device manufactures and dispenses the finished product, ready for use, without any direct human handling of chemicals (as shown in Figure A1).

### Automated Solution Dispenser:

Automated Solution Dispenser relies on the technology of mixing and preparing ready-to-use products, facilitating the acquisition of laboratory results for sample analysis. This approach significantly saves time and effort without using the chemical principles of manufacturing. The functions of the Automated Solution Dispenser include measuring, dispensing, mixing, pH adjustment, temperature control, degassing, filtering, filling, labeling, and cleaning before or after solution preparation, etc.

### Mobile Detergent Manufacturing Factory:

It relies on the technology of manufacturing and chemical compounding, starting from combining raw materials in variable chemical proportions based on the type and quantity of the desired product. The device determines and combines these proportions based on chemical formulas inputted through the programming language used during its manufacturing. Upon the user selecting the type and quantity of the required detergent, the device manufactures and dispenses the finished product, ready for use, without any direct human handling of the initial raw chemicals. This prevents any potential errors during the product manufacturing process. The technology of the Mobile Factory is based on reducing chemical waste harmful to humans and the environment, producing only the actual amount the consumer needs, reducing financial waste. Furthermore, the functions of Mobile Factory include identifying problem types, selecting the appropriate solution, self-cleaning, identifying raw materials before use, preventing the combination of any chemicals incompatible with the product based on the consumer's request, compounding and preparation using industrial methods suitable for the chemicals used, determining the correct dosage of each raw chemical involved in the manufacturing process, displaying instructions for correct product usage through video clips within the device's associated mobile application, and suggesting helpful cleaning tools such as brushes and towels.

Based on the foregoing, it can be concluded that the Automated Solution Dispenser utilizes ready-made products, whereas the Mobile Detergent Manufacturing Factory relies on manufacturing from chemical compounds to produce finished products. The difference between the Mobile Detergent Manufacturing Factory and the Automated Solution Dispenser lies in their objectives and functionality, and the benefits derived from each. The Mobile Detergent Manufacturing Factory is classified as an industrial device intended for manufacturing, starting from the stage of chemical compounds to reach finished products. This is evidenced by the production lines of detergent factories producing large commercial quantities, while also considering the provision of the correct dosage concept, safe usage, and meeting the consumer's actual need through the Mobile Detergent Manufacturing Factory. On the other hand, the Automated Solution Dispenser is classified as a medical device for analyzing samples, intended to save time and effort in obtaining laboratory results by mixing and preparing the necessary solutions for testing and analyzing the samples inputted into the device.

### Advantages of the Invention:

The invention features a small, hand-held, easily portable industrial system for the purpose of proper manufacture and production of household cleaning agents, without human intervention, unlike current market practices. It is worth noting that consumers currently rely on ready-made products, requiring them to check expiration dates for efficacy and adhere to proper storage methods to ensure optimal performance. This limits consumers to the solutions offered by detergent manufacturers, often not addressing their specific needs. Consequently, this leads consumers to search online for solutions and experiments, even though some solutions require specific manufacturing conditions suited to the chemicals involved. Consumers also need storage space for ready-made products, as is the case with currently available products. However, the Mobile Detergent Manufacturing Factory produces detergents on demand and in the quantity needed by the consumer, increasing product efficacy due to on-the-spot production. It also eliminates the need for large storage spaces as it only produces the quantity needed by the consumer. The device information is also updated regularly to address evolving consumer needs, providing solutions without risky experimentation or improper chemical handling.

To illustrate, some consumers mix different cleaning agents to address issues like rust, stains or grease, resulting in a completely different substance that not only fails to resolve the intended issue but may also cause health problems due to the chemical reaction, as proper manufacturing and chemical compounding principles are disregarded. This practice is extremely dangerous. Furthermore, some consumers store products improperly, diminishing their effectiveness and potency. The Mobile Detergent Manufacturing Factory solves this by producing detergents on demand, ensuring higher efficacy and potency. The invention's vision also includes using eco-friendly raw materials to protect human health and provide solutions for household problems like various stains, rust, grease, paint, and regular cleaning, catering to all consumer needs.

### Advantages of the Device:

- Small size and portable as a mobile factory with production capacity suitable for consumer needs. Saves storage space. Ability to produce the actual consumer need on the spot without the need for the consumer to keep chemicals continuously in their home, which reduces human exposure to chemicals, whether liquid or volatile. The device ensures the correct dosage is used, minimizing errors and chemical contact with humans. Solves most environmental problems professionally without human intervention in mixing, dilution, or misuse of chemicals. Reduces chemical waste to preserve human and environmental health by using eco-friendly materials, and in case of consumer contact with products, they will be safe. Reduces financial waste, as the cost of the product will be restricted to the manufacturing cost incurred by the consumer. The device helps identify environmental problems and provide quick solutions. Easy to operate and interact with the device through electronic applications, which contain the operating and production manual in addition to home first aid methods in case of misuse of products by the consumer.

### Brief Description of the Drawings:

1. Figure A1: External Image of the Device.
2. Figure 2: Main Circuit.
3. Figure 3: Connection between Device Elements.
4. Figure 4: Device Elements.
5. Figure 5: An Image of the Codes used in the Programming Language.

### Detailed Description:

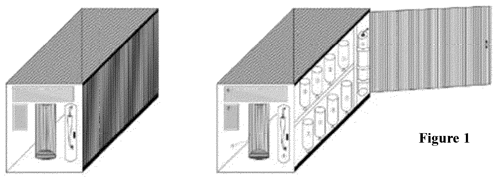

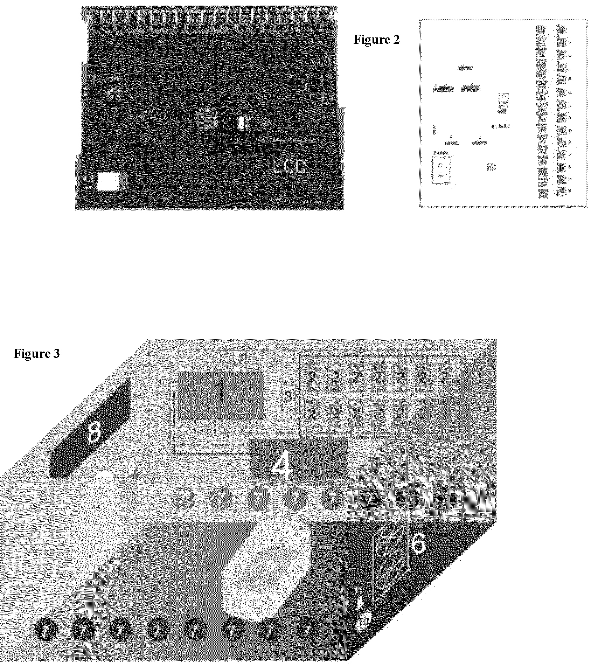

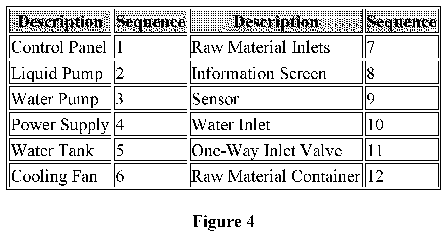

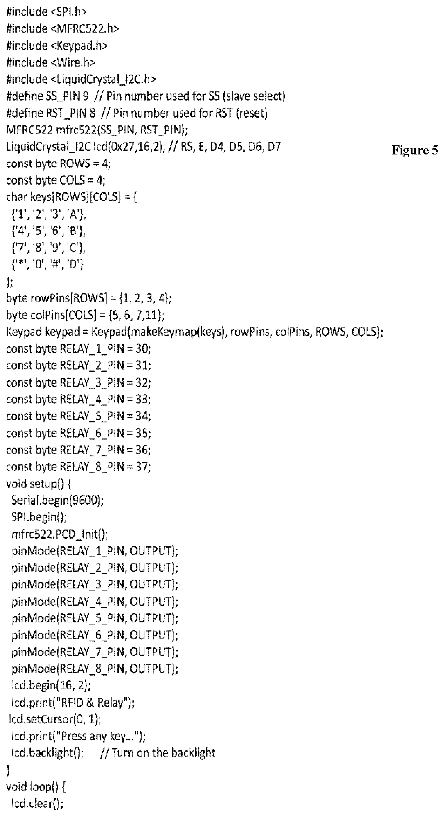

The process begins with the consumer selecting the desired product by choosing its name and image or by specifying the type of cleaning problem via the display screen (as shown in Figure 4-8 below) or through the device's mobile application. After the consumer selects the desired product, the QR code on the container intended for the finished product is scanned through a sensor reader to verify that the container matches the consumer's request (as shown in Figure 4-9) in order to prevent any errors. If an incorrect reading occurs, an error message appears on the screen, guiding the consumer to the correct procedure. A signal is also sent to the main circuit board to halt the process until the correct container is placed. The command is sent from the screen to the main circuit board (shown in Figure 4-1; a complete circuit diagram is shown in Figure 2). The circuit board then sends a control command to activate the liquid pumps (Figure 4-2), which are programmatically linked to the chemical compound containers for the product requested by the consumer. The raw materials are drawn through the raw material container outlets (Figure 4-7). The pumps draw the raw materials from their designated containers (Figure 4-12) and dispense them into the container provided by the consumer. All raw chemical materials are in liquid form, facilitating their dissolution in water. Upon contact with water, the liquid raw materials dissolve immediately, forming the desired cleaning agent. The water tank is filled from an external water source through a non-return valve (Figure 4-5). A control command then activates the water pump (Figure 4-3) to draw water from the tank (Figure 4-5) according to pre-programmed chemical proportions and methods based on the type of product requested and the chemicals used. The pump then injects the water into the final product container through a valve (Figure 4-10) to complete the manufacturing process. The electrical components are powered by a power supply (Figure 4-4), which is connected to all electrical elements. The device is equipped with cooling fans (Figure 4-6), which activate automatically when the device is plugged in to cool the components and the device itself. Figure 3 below illustrates the connections between the device parts.

### Device Operation:

After understanding the electrical, mechanical and software components of the device and their functions, the device is placed in the location of the consumer's choosing, away from children. The consumer connects the device to a power source and a water supply. Then, the containers for the raw materials are installed in their correct designated locations. The consumer then goes to the display screen, turns on the device, and selects the appropriate language (all major languages are available). They then specify the type of request, either a specific cleaning problem requiring a product or a product for general cleaning. The device receives the command and displays information about the solution or the product on the screen to confirm the consumer's selection. After confirmation, the device begins mixing the materials and manufacturing the requested product. During this process, a message appears on the screen or the smart device, providing the consumer with instructions for the proper use of the product and recommending suitable cleaning tools such as brushes or towels. The consumer then receives the detergent container from the device to begin cleaning.

## Claims

1. The process of manufacturing (household cleaning agents) products is as follows:
A. The desired cleaning agent is requested either through the product information screen (Figure 4-8) or via a mobile device application electronically connected to the control panel (Figure 4-1).
B. A command is sent to the containers holding the raw materials (Figure 4-12). These containers are connected to liquid pumps (Figure 4-2), which transfer the raw materials in specific proportions through shared tubes to manufacture the requested cleaning agent. The manufacturing process depends on the components of each cleaning agent, the role of each raw material, its reaction method, and the precise timing for adding each material. The materials are added gradually. Liquid raw materials are used to facilitate the manufacturing process. These are pumped at varying speeds to control the interaction of multiple raw materials involved in creating a specific cleaning agent. Upon mixing within the tubes, the desired chemical reaction occurs, forming the cleaning agent.

2. The cleaning agent manufacturing device is equipped with an electrical system that includes:
An information screen (Figure 4-8) connected to the control panel (Figure 4-1) to receive initial commands. The control panel then sends commands to the mechanical parts containing the raw material containers (Figure 4-12), a connected water tank (Figure 4-5), and a water pump (Figure 4-3). Distilled water is used in the manufacturing process.
Cooling fans (Figure 4-6) to cool the control panel and internal components from the heat generated by the chemical reactions.
A sensor for reading product codes to facilitate the manufacturing process and reduce errors.
At least one one-way valve for pumping the required raw materials in specific quantities, with a non-return feature.

3. The device includes a software component connected to an application that runs on mobile devices. This allows remote control of the manufacturing process and enables users to share cleaning problems with the device for providing solutions in the form of cleaning agents.

4. The device utilizes wireless technology.

5. The software used in the device includes:
a. Chemical formulations.
b. Stain identification using tablet cameras linked to the software. This allows for identification of stain types from images and suggests the optimal cleaning product.
The desired cleaning agent is inputted through the control panel (either on the device itself or a mobile device). The raw materials for the requested cleaning agent are passed through tubes connected to the consumer's cleaning agent container. The transfer is controlled based on each raw material's role in the manufacturing process, utilizing the fluidity of the raw materials and their interaction during transfer. This results in the desired chemical reaction to form the cleaning agent.
